# EUROPEAN PATENT SPECIFICATION

(11) **EP 1 951 328 B1**
(45) Date of publication and mention of the grant of the patent: **05.03.2014**
(21) Application number: 06812242.3
(22) Date of filing: 27.10.2006
(51) Int. Cl.: A61L 26/00, A61L 15/28

(54) **SUSTAINED RELEASE FILM FORMULATION FOR HEALING WOUND COMPRISING EPIDERMAL GROWTH FACTOR**
FILM FORMULIERUNG MIT VERZÖGERTER FREISETZUNG ZUR WUNDHEILUNG MIT EPIDERMALEM WACHSTUMSFAKTOR
FORMULATION DE FILM DE LIBÉRATION PROLONGEE POUR CICATRISATION DE PLAIE COMPRENANT UN FACTEUR DE CROISSANCE ÉPIDERMIQUE

(30) Priority: 14.11.2005 KR 20050108344
(43) Date of publication of application: 06.08.2008
(73) Proprietor: Daewoong Co., Ltd., Sungnam-si, Kyunggi-do 462-120 (KR)
(72) Inventor: KIM, Sun Hee, Chungcheongbuk-do 360-181 (KR); LEE, Sang Kil, Gyeonggi-do 440-847 (KR); LEE, Min Suk, Seoul 137-933 (KR); HONG, Joon Pio, Seoul 138-878 (KR)
(74) Representative: Wichmann, Hendrik
(86) International application number: PCT/KR2006/004401
(87) International publication number: WO 2007/055481

(56) References cited:
- EP-A1- 0 312 208
- EP-A2- 0 267 015
- WO-A1-01/62276
- JP-A- 2000 167 036
- US-A- 5 840 338
- US-A- 6 124 273
- US-A1- 2003 203 011
- US-B1- 6 395 029

## Description

### TECHNICAL FIELD

The present invention relates to a sustained release film formulation for healing wound comprising epidermal growth factor; chitosan as defined in the claims; one or more viscosity modifiers selected from the group consisting of hydroxypropylmethylcellulose as defined in the claims, gellan gum and pullulan; one or more plasticizers selected from the group consisting of glycerin, propylene glycol, polyethylene glycol, polyvinyl alcohol and polyvinylpyrrolidone; and one or more antioxidants selected from the group consisting of EDTA and vitamin C.

### BACKGROUND ART

Epidermal growth factor is polypeptide whose molecular weight is 6045, having three disulfide bonds and fifty-three (53) amino acids known as urogastrone. The factor is known to have such activities as mitosis promotion and cell growth promotion of various cells including epidermal cell and mesenchymal cell, inhibition of gastric acid secretion, etc., and so be effective for treating skin or cornea wound, or gastric ulcer (Carpenter, Experimental Cell Research, 164:1-10, 1986).

However, epidermal growth factor is too unstable at room temperature, particularly, in the presence of water. And, the latent period of 8 to 12 hrs is required in order to induce cells to synthesize DNA at wound site. However, the latent period of epidermal growth factor is about 1 hr or less, which is too short to achieve desired effect. Also, the epidermal growth factor is difficult to apply to external skin wound since the factor is denaturalized, decomposed, condensed and precipitated by proteolytic enzyme existing at wound site when applied to skin, and so loses biological activity (Manning et al., Pharmaceutical Res.,: 903-917, 1989).

To overcome biological instability among the above drawbacks of epidermal growth factor, and sufficiently maintain therapeutic effect, it was reported that an effective concentration of epidermal growth factor should be consistently maintained at wound site (Franklin et al., J. Lab. Clin. Med., 108:103-108, 1986). Thus, many studies have been made to develop a sustained release formulation which can consistently provide epidermal growth factor into wound site. USP No. 4,944,948 discloses epidermal growth factor/liposome gel formulation which consistently provides epidermal growth factor to wound site by using neutral phospholipids, negatively charged phospholipids, and cholesterol. In particular, this invention provides a method for consistently releasing epidermal growth factor for 12 hrs or more, but the method has limits in attaining storage stability, and so is difficult to apply in the industry. Therefore, to show a sufficient therapeutic effect, epidermal growth factor as drug should retain consistent drug-releasing property, with maintaining biological activity and physicochemical stability related to constant purity and content, under long term storage.

Also, EP No. 267015 discloses a film prepared by using epidermal growth factor with hydrophilic cellulose polymer, particularly, with hydroxypropylmethylcellulose used in the present invention. However, the film has a problem that the effect of epidermal growth factor is not significant enough to heal wound.

EP 0 312 208 A1 discloses an aqueous gel formulation for topical and incisional wound healing, comprising:
a) an effective wound healing amount of a polypeptide growth factor having human mitogenic or angiogenic activity; and
b) a water soluble or water swellable, pharmaceutically acceptable polymer for providing viscosity within the range 1,000 to 12,000,000 cps at room temperature.

US 6,124,273 A discloses a composition of matter that provides sustained release of proteins, said composition comprising a covalently cross-linked N,O-carboxymethyl chitosan hydrogel having a protein to be delivered incorporated therein in sufficient concentration to provide said sustained release.

WO 01/62276 A1 discloses a stable composition which comprises a biologically active epidermal growth factor (hereinafter referred to as"EGF") as an active ingredient and a carboxyvinyl polymer as a base.

### DETAILED DESCRIPTION OF THE INVENTION

### TECHNICAL SUBJECT

The object of the present invention is to provide a sustained release film formulation for healing wound comprising epidermal growth factor which can consistently release main ingredient for 1 week, with maintaining the biological activity and physicochemical stability of epidermal growth factor, by using chitosan as defined in the claims having antibacterial activity as main base for maximizing would-healing effect of epidermal growth factor; hydroxypropylmethylcellulose as defined in the claims, gellan gum, or pullulan as film base for consistently releasing main ingredient with maintaining stability of epidermal growth factor; antioxidant which is EDTA, and/or vitamin C as stabilizing agent; and one or more of glycerin, propylene glycol, polyvinyl alcohol, polyvinylpyrrolidone, polyethylene glycol as plasticizer.

When the film according to the present invention is applied to wound site, the dried film absorbs exudation to be changed to hydrogel, and so can be easily attached to living body. Also, the film can maximize the therapeutic effect of epidermal growth factor, and can keep the effective therapeutic concentration only by one application a day since the main base, chitosan, itself has antibacterial activity.

### BRIEF DESCRIPTION OF THE DRAWINGS

Figs. 1 to 3 are graphs showing *in vitro* release rate of the film for healing wound comprising epidermal growth factor and chitosan according to the present invention, for one day.
Fig. 4 is a graph showing production rates of impurities of the film for healing wound comprising epidermal growth factor and chitosan according to the present invention, under the cold-storage condition for a year.
Figs. 5 and 6 each are photographs of wound site before and after treatment of the control group, and the film for healing wound comprising epidermal growth factor and chitosan according to the present invention.

### MODE FOR CARRYING OUT THE INVENTION

The present invention relates to a sustained release film formulation for healing wound comprising epidermal growth factor; chitosan as defined in the claims; one or more viscosity modifiers selected from the group consisting of hydroxypropylmethylcellulose as defined in the claims, gellan gum and pullulan; one or more plasticizers selected from the group consisting of glycerin, propylene glycol, polyethylene glycol, polyvinyl alcohol and polyvinylpyrrolidone; and one or more antioxidants selected from the group consisting of EDTA and vitamin C

And, the present invention relates to a film formulation as defined in the claims characterized in additionally comprising foam type of dressing or hydrocolloidal type of dressing to the above sustained release film formulation.

The epidermal growth factor which is an active ingredient of the sustained release film formulation of the present invention can be applied to either natural protein or recombination protein. The epidermal growth factor in the present film formulation is used in an amount of 0.1 to 10,000µg/g, more preferably 1 to 5,000µg/g which is a pharmacologically effective concentration. If the content of epidermal growth factor is less than 0.1µg/g, the film formulation is not pharmacologically effective. If the content is more than 10,000µg/g, the film formulation may be toxic. Here, the pH of the film formulation is preferably adjusted to 5 to 7, which is the range to maintain the stability of epidermal growth factor.

Chitosan which is the main base or the present invention is according to the present invention a polymer having 10 to 150 kDa of molecular weight. If the molecular weight is smaller than 10kDa, chitosan cannot keep the viscosity as hydrogel. If the molecular weight is more than 150kDa, the viscosity of polymer solution is so high that the preparation is very difficult. Also, chitosan in the film formulation is used in an amount of 1.0 to 10.0 w/w% based on the total weight. If the amount of chitosan is out of said range, the viscosity is so low that the preparation is difficult.

Antioxidant used for stabilizing epidermal growth factor is one or more selected from the group consisting of EDTA and vitamin C. In the present invention, antioxidant is preferably used in an amount of 0.01 to 10 w/w% based on the total weight, more preferably, 0.05 to 5 w/w%. If the amount is out of said range, it is difficult to achieve the stability of epidermal growth factor. Also, preferably, the present film formulation may additionally comprise one or more buffers selected from the group consisting of sodium hydrogen phosphate, sodium hydrogen carbonate, and sodium citrate; or one or more amino acids selected from the group consisting of L-histidine, L-lysine and L-arginine for stabilizing the formulation.

In the present invention, viscosity modifiers is one or more selected from the group consisting of hydroxypropylmethylcellulose as defined in the claims, gellan gum and pullulan. Here, hydroxypropylmethylcellulose may be classified into low-viscosity hydroxypropylmethylcellulose and high-viscosity hydroxypropylmethylcellulose according to viscosity. The low-viscosity hydroxypropylmethylcellulose functions as a film auxiliary base, and the high-viscosity hydroxypropylmethylcellulose plays a role to control sustained release. According to the invention, low-viscosity hydroxypropylmethylcellulose of 10 to 100 cps and high-viscosity hydroxypropylmethylcellulose of 1,000 to 100,000 cps are used in mixture. The viscosity modifier is preferably used in an amount of 0.01 to 20.0 w/w%, more preferably, 0.01 to 5 w/w%. If the amount is out of said range, the viscosity of base before drying is affected enough to cause a problem in the preparation.

The sustained release film formulation of the present invention additionally comprises one or more plasticizers selected from the group consisting of glycerine, polypropylene glycol, polyvinylpyrrolidone and polyethylene glycol. The amount of plasticizer is preferably 1 to 40 w/w%, more preferably, 1 to 20 w/w%. If the amount of plasticizer is less than 1 w/w%, the film would get loose from the coating paper. If the amount is more than 40 w/w%, the drying time and total weight are increased to facilitate gelation, which may cause a problem during the process.

To the sustained release film formulation of the present invention, water is added to make the total volume 100 w/w%.

Foam type of dressing or hydrocolloidal type of dressing known in the art may be added to the film formulation according to the present invention. Also, to keep the film shape and prevent external pollution, a dressing additionally comprising silver or alginate may be added to foam type of dressing or hydrocolloidal type of dressing.

The sustained release film formulation for healing wound according to the present invention may be prepared by the following methods.

Chitosan as defined in the claims, a viscosity modifier, and a plasticizer are put into a suitable container. Then, distilled water is added thereto, and stirred to be completely homogenized. Antioxidant as a stabilizer, and epidermal growth factor are added thereto, and homogeneously mixed to prepare a composition for preparing the present film. Here, the viscosity of the composition is preferably 1,000 to 150,000 cps, more preferably, 4,000 to 120,000 cps. If the viscosity is out of said range, the preparation itself would be difficult because the casting is difficult.

The composition as prepared above is kept in a refrigerator of 4∼10°C for about a day, and then is cast onto coated paper, and dried to prepare the sustained release film formulation for healing wound. The thickness of the film prepared above is preferably 0.001 to 1 mm which is the range easy for the preparation and treatment, more preferably, 0.01 to 0.5 mm. The content of water in the film, which is an important factor to affect the stability of epidermal growth factor and the preparation condition of film, is preferably 1 to 30 %, more preferably, 10 to 25 %.

Hereinafter, the present invention is specifically described by examples. However, the examples are provided to explain the present invention, and so the scope of the present invention is not limited by them in any manner.

### <Example 1: Preparation of the sustained release film formulation according to the present invention>

The sustained release film formulation of the present invention comprising epidermal growth factor, chitosan, viscosity modifier, plasticizer and stabilizer was prepared by the following process:
Chitosan, hydroxypropylmethylcellulose (HPMC), one or more viscosity modifiers selected from the group consisting of gellan gum and pullulan, and one or more plasticizers selected from the group consisting of glycerin, polypropylene glycol, polyvinylpyrrolidone (PVP), and polyethylene glycol (PEG) were put into a suitable container. Then, distilled water was added thereto, and stirred to be completely homogenized. One or more antioxidants selected from the group consisting of EDTA and vitamin C as stabilizer, and recombinant epidermal growth factor, i.e., lyophilized epidermal growth factor formulation (Daewoong Co.), were added thereto and homogeneously mixed. Then, the mixture was kept in a refrigerator of 4∼10°C for about a day, and then was cast onto coated paper, and ventilated or lyophilized to prepare the sustained release film formulation for healing wound according to the present invention.

### <Example 2: Analysis of physicochemical property of the sustained release film formulation according to the present invention by ingredients>

To analyze the physicochemical property of the sustained release film formulation according to the present invention by ingredients, films (DWF1 to DWF6) comprising epidermal growth factor were prepared by using the ingredients described in the following Table 1 according to the same method as Example 1. Only, the thickness of films was 1.0mm when the film is cast, and the method of dryness was ventilation at 40°C for 1 hr.

**[Table 1]**

| Composition | Components (in 10g) | |
|---|---|---|
| DWF 1 (Comparative Example) | epidermal growth factor | 1mg |
| | chitosan | 1g |
| | glycerine | 109 |
| | water | proper quantity |
| DWF2 (Comparative Example) | epidermal growth factor | 1mg |
| | chitosan | 1g |
| | hydroxypropylmethylcellulose 50 cps | 10g |
| | glycerine | 10g |
| | water | proper quantity |
| DWF3 | epidermal growth factor | 1mg |
| | chitosan | 1g |
| | hydroxypropylmethylcellulose 50 cps | 5g |
| | hydroxypropylmethylcellulose 4000cps | 1g |
| | glycerine | 10g |
| | water | proper quantity |
| DWF4 (Comparative Example) | epidermal growth factor | 1mg |
| | chitosan | 1g |
| | gelatin | 10g |
| | glycerine | 10g |
| | water | proper quantity |
| DWF5 (Comparative Example) | epidermal growth factor | 1 mg |
| | chitosan | 1g |
| | polyethylene glycol | 10g |
| | glycerine | 10g |
| | water | proper quantity |
| DWF6 (Comparative Example) | epidermal growth factor | 1mg |
| | chitosan | 1g |
| | polyvinylpyrrolidone | 10g |
| | glycerine | 10g |
| | water | proper quantity |

The purity of epidermal growth factor in DWF1 to DWF6 prepared above was determined by reverse phase HPLC. And, the property and condition, thickness, and moisture content of the film prepared above were measured, and the results were shown in the following Table 2.

**[Table 2]**

| Classification | Property and condition | Thickness | Moisture content (%) | Purity of EGF (%) |
|---|---|---|---|---|
| DWF1 (chitosan alone) | shrinking | 50 ± 10µm | 20 ± 5% | 92 ± 0.5% |
| DWF2 ((HPMC 50cps) | a little shrinking | 100 ± 10µm | 20 ± 2% | 94 ± 0.5 % |
| DWF3 (HPMC 50 cps / HPMC 4000 cps) | good | 150 ± 10µm | 20 ± 3% | 95 ± 0.6% |
| DWF4 (gelatin) | good | 140 ± 10µm | 20 ±4 % | 87 ± 0.9% |
| DWF5 (PEG) | good | 100 ± 10µm | 20 ± 5% | 93 ± 0.5 % |
| DWF6 (PVP) | good | 100 ± 10µm | 20 ± 3% | 93 ± 0.6% |

As shown in the above Table 2, it was confirmed that the films prepared by mixing other polymers such as HPMC, glycerine, PEG, and PVP (DWF2-DWF6) have more superior property and condition than DWF1 film prepared by chitosan only. Only, when gelatin was used as polymer (DWF4), the epidermal growth factor was degraded to result in decreasing the purity of epidermal growth factor during the process of increasing the temperature at the time of preparing the film base. In particular, when hydroxypropylmethylcellulose (HPMC) was used as a viscosity modifier, the epidermal growth factor was most superior in the property and condition, and purity.

### <Example 3: Release property of epidermal growth factor according to viscosity and thickness of film>

To confirm the release property of epidermal growth factor according to the viscosity of film and the contents of main ingredients, the films (DWF7 to DWF14) were prepared by using the ingredients described in the following Table 3 according to the same methods as Example 1 and Example 2. DWF13 and DWF14 films were prepared in different casting thicknesses, and the viscosity and thickness of each film were measured and shown in the following Table 3.

**[Table 3]**

| Composition | Components (in 10g) | Content | Viscosity | Thickness |
|---|---|---|---|---|
| DWF7 | epidermal growth factor | 10.0 mg | 8~10mps | 160 ± 10µm |
| | chitosan | 5.0 g | | |
| | hydroxypropylmethylcellulose 50 cps | 10.0 g | | |
| | hydroxypropylmethylcellulose 4000cps | 1.0 g | | |
| | pullulan | 0.05 g | | |
| | glycerine | 10.0 g | | |
| | distilled water | proper quantity (in 100g) | | |
| DWF8 | epidermal growth factor | 10.0 mg | 12∼15mps | 160 ± 10µm |
| | chitosan | 5.0 g | | |
| | hydroxypropylmethylcellulose 50 cps | 10.0 g | | |
| | hydroxypropylmethylcellulose 4000cps | 1.0 g | | |
| | pullulan | 0.1 g | | |
| | glycerine | 10.0 g | | |
| | distilled water | proper quantity (in 100g) | | |
| DWF9 | epidermal growth factor | 10.0 mg | 7~9mps | 155 ± 10µm |
| | chitosan | 5.0 g | | |
| | hydroxypropylmethylcellulose 50 cps | 10.0 g | | |
| | hydroxypropylmethylcellulose 4000cps | 0.5 g | | |
| | pullulan | 0.05 g | | |
| | glycerine | 10.0 g | | |
| | distilled water | proper quantity (in 100g) | | |
| DWF10 | epidermal growth factor | 10.0 mg | 17∼20mps | 165 ± 10µm |
| | chitosan | 5.0 g | | |
| | hydroxypropylmethylcellulose 50 cps | 10.0 g | | |
| | hydroxypropylmethylcellulose 4000cps | 2.0 g | | |
| | pullulan | 0.05 g | | |
| | glycerine | 10.0 g | | |
| | distilled water | proper quantity (in 100g) | | |
| DWF11 | epidermal growth factor | 5.0 mg | 8∼10mps | 160 ± 10µm |
| | chitosan | 5.0 g | | |
| | hydroxypropylmethylcellulose 50 cps | 10.0 g | | |
| | hydroxypropylmethylcellulose 4000cps | 1.0 g | | |
| | pullulan | 0.05 g | | |
| | glycerine | 10.0 g | | |
| | distilled water | proper quantity (in 100g) | | |
| DWF12 | epidermal growth factor | 1.0 mg | 8∼10mps | 160 ± 10µm |
| | chitosan | 5.0 g | | |
| | hydroxypropylmethylcellulose 50 cps | 10.0 g | | |
| | hydroxypropylmethylcellulose 4000cps | 1.0 g | | |
| | pullulan | 0.05 g | | |
| | glycerine | 10.0 g | | |
| | distilled water | proper quantity (in 100g) | | |
| DWF13 (casting of 2.0mm) | epidermal growth factor | 10.0 mg | 12∼15mps | 300 ± 10µm |
| | chitosan | 6.25 g | | |
| | hydroxypropylmethylcellulose 50 cps | 12.5 g | | |
| | hydroxypropylmethylcellulose 4000cps | 1.25 g | | |
| | pullulan | 0.125 g | | |
| | glycerine | 12.5 g | | |
| | distilled water | proper quantity (in 125g) | | |
| DWF14 (casting of 3.0mm) | epidermal growth factor | 10.0 mg | 12∼15mps | 455 ± 10µm |
| | chitosan | 7.5 g | | |
| | hydroxypropylmethylcellulose 50 cps | 15.0 g | | |
| | hydroxypropylmethylcellulose 4000cps | 1.5 g | | |
| | pullulan | 0.15 g | | |
| | glycerine | 15.0 g | | |
| | distilled water | proper quantity (in 150g) | | |

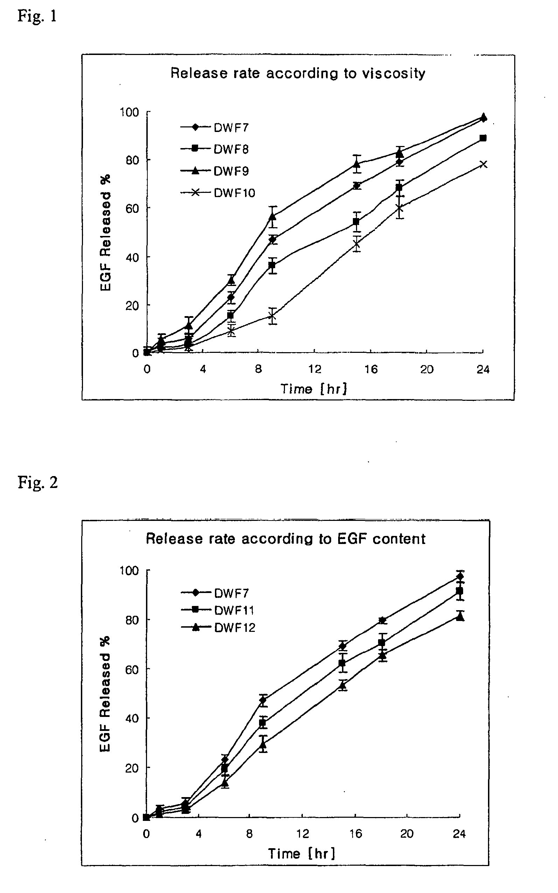

To analyze each release amount of epidermal growth factor from DWF7 to DWF12 films prepared above, the release test was conducted. The results were shown in the following Table 4 and Figs. 1 and 2.

The release test was conducted by using Franz diffusion apparatus. The release medium was filled with phosphoric acid buffer (pH 7.4, 0.02% Tween 20), kept at 32 ± 0.5°C, and set to directly contact to buffer without membrane. Thus prepared films were cut to the test size for application. The mixtures of 300µℓ each were taken from the films at the designated times of 1, 3, 6, 9, 15, 18 and 24 hrs. Then, the release amounts of EGF were determined by HPLC, and the determined values were shown as release % to initial value.

**[Table 4]**

| Time (hr) | Release amount of epidermal growth factor % (mean ± standard deviation) | | | | | |
|---|---|---|---|---|---|---|
| | DWF7 | DWF8 | DWF9 | DWF10 | DWF11 | DWF12 |
| 1 | 3.5 ± 1.25 | 2.3 ± 1.23 | 5.4 ± 2.35 | 0.9 ± 0.20 | 2.1 ± 0.91 | 1.2 ± 0.09 |
| 3 | 5.7 ± 2.34 | 3.4 ± 1.10 | 11.3 ± 2.43 | 2.1 ± 1.12 | 4.3 ± 1.25 | 3.2 ± 1.20 |
| 6 | 23.0 ± 2.24 | 15.2 ± 3.2 | 30.2 ± 3.50 | 9.2 ± 1.26 | 19.2 ± 2.30 | 14.1 ± 2.34 |
| 9 | 47.0 ± 2.53 | 36.2 ± 2.35 | 56.3 ± 2.26 | 15.2 ± 2.35 | 38.2 ± 2.5 | 29.3 ± 3.23 |
| 15 | 69.2 ± 1.92 | 54.3 ± 3.26 | 78.2 ± 4.30 | 45.3 ± 3.24 | 62.1 ± 3.63 | 53.2 ± 2.12 |
| 18 | 79.3 ± 1.20 | 68.2 ± 4.24 | 83.2 ± 3.64 | 60.2 ± 3.24 | 70.21 ± 4.24 | 65.3 ± 2.51 |
| 24 | 97.5 ± 2.10 | 89.3 ± 3.24 | 98.1 ± 2.12 | 78.2 ± 4.53 | 91.2 ± 3.40 | 81.3 ± 1.92 |

As shown in Table 4 and Fig. 1, it was confirmed that the higher the viscosity of film is, the more the release of epidermal growth factor is delayed. Also, as shown in Fig. 2, it was confirmed that the release amount of epidermal growth factor from DWF12 (1.0mg) containing the smallest amounts of main ingredients were less than those from DWF11 (5.0mg), whereby it was concluded that the smaller the amounts of main ingredients are, the more the release is delayed. This confirmed that the release rate of epidermal growth factor is controlled by the viscosity and amount, and so the epidermal growth factor shows linear release for 24 hrs.

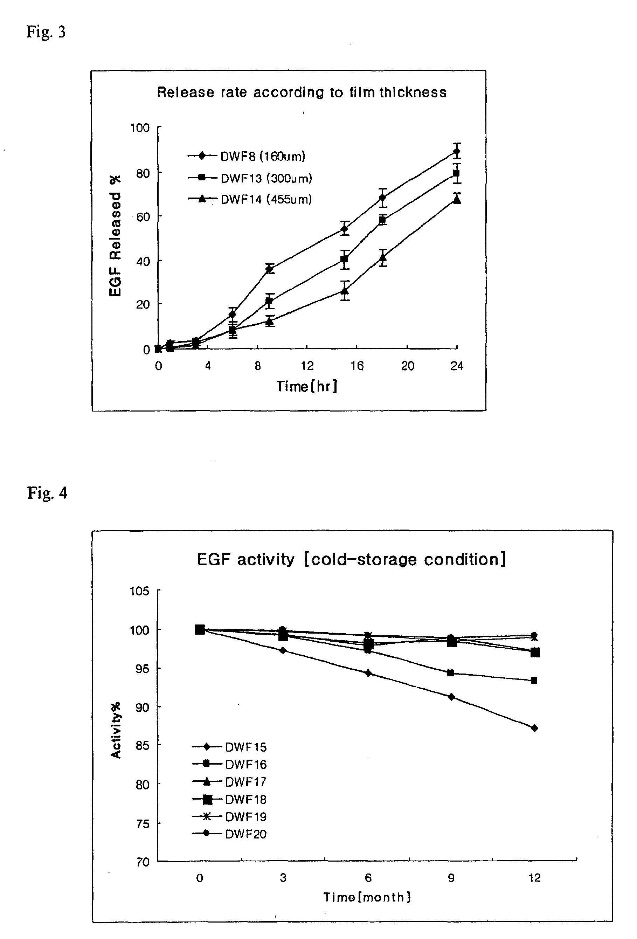

Also, the release amounts of epidermal growth factor from DWF13 and DWF14 were measured, and shown in the following Table 5 and Fig. 3. As shown in Table 5, it was confirmed that the thicker the film is, the more the release rate of epidermal growth factor is delayed. In sum, it was confirmed that the release rate of epidermal growth factor is controlled by the viscosity and thickness of film.

**[Table 5]**

| Time (hr) | Release amount of epidermal growth factor % (mean ± standard deviation) | | |
|---|---|---|---|
| | DWF8 (160 ± 10*µ*m) | DWF13 (300 ± 10*µ*m) | DWF14 (455 ± 10*µ*m) |
| 1 | 2.3 ± 1.23 | 0.3 ± 0.23 | 0.4 ± 0.35 |
| 3 | 3.4 ± 1.10 | 2.4 ± 1.10 | 1.3 ± 1.43 |
| 6 | 15.2 ± 3.2 | 8.2 ± 3.8 | 8.2 ± 2.50 |
| 9 | 36.2 ± 2.35 | 21.2 ± 3.35 | 12.3 ± 2.26 |
| 15 | 54.3 ± 3.26 | 40.3 ± 4.26 | 26.2 ± 4.30 |
| 18 | 68.2 ± 4.24 | 58.2 ± 2.24 | 41.2 ± 3.64 |
| 24 | 89.3 ± 3.24 | 79.3 ± 4.24 | 68.1 ± 2.12 |

### <Example 4: Stability of epidermal growth factor in the film according to stabilizer addition>

To confirm the stability of epidermal growth factor in the sustained release film formulation of the present invention according to the addition of stabilizer, the content and activity of epidermal growth factor by time were measured from the films comprising epidermal growth factor, chitosan, viscosity modifier and plasticizer for each case that stabilizer is not added (DWF 15); only antioxidant is added (DWF 17 and 18); only amino acid is added (DWF 16); antioxidant and amino acid are added (DWF 19); and antioxidant and buffer are added (DWF 20).

For this experiment, DWF15 to DWF20 films were prepared by using the ingredients described in the following Table 6 according to the same methods as Example 1 and Example 2.

**[Table 6]**

| Composition | Components (in 100g) | | Film thickness |
|---|---|---|---|
| DWF15 (-) | epidermal growth factor | 1.0mg | 160 ± 10 *µ*m |
| | chitosan | 5.0g | |
| | hydroxypropylmethylcellulose 50 cps | 10.0g | |
| | hydroxypropylmethylcellulose 4000cps | 1.0g | |
| | pullulan | 0.05g | |
| | glycerine | 10.0g | |
| | distilled water | proper quantity | |
| DWF16 (L-histidine) | epidermal growth factor | 1.0mg | 160 ± 10 *µ*m |
| | chitosan | 5.0g | |
| | hydroxypropylmethylcellulose 50 cps | 10.0g | |
| | hydroxypropylmethylcellulose 4000cps | 1.0g | |
| | pullulan | 0.05g | |
| | glycerine | 10.0g | |
| | L-histidine | 0.1 g | |
| | distilled water | proper quantity | |
| DWF17 (EDTA) | epidermal growth factor | 1.0mg | 160 ± 10 *µ*m |
| | chitosan | 5.0g | |
| | hydroxypropylmethylcellulose 50 cps | 10.0g | |
| | hydroxypropylmethylcellulose 4000cps | 1.0g | |
| | pullulan | 0.05g | |
| | glycerine | 10.0g | |
| | EDTA Disodium | 0.05g | |
| | distilled water | proper quantity | |
| DWF18 (ascorbic acid) | epidermal growth factor | 1.0mg | 160 ± 10 *µ*m |
| | chitosan | 5.0g | |
| | hydroxypropylmethylcellulose 50 cps | 10.0g | |
| | hydroxypropylmethylcellulose 4000cps | 1.0g | |
| | pullulan | 0.05g | |
| | glycerine | 10.0g | |
| | ascorbic acid | 0.05g | |
| | distilled water | proper quantity | |
| DWF19 (EDTA + L-histidine) | epidermal growth factor | 1.0mg | 160 ± 10 *µ*m |
| | chitosan | 5.0g | |
| | hydroxypropylmethylcellulose 50 cps | 10.0g | |
| | hydroxypropylmethylcellulose 4000cps | 1.0g | |
| | pullulan | 0.05g | |
| | glycerine | 10.0g | |
| | EDTA Disodium | 0.05g | |
| | L-histidine | 0.1g | |
| | distilled water | proper quantity | |
| DWF20 (EDTA + Phosphate buffer) | epidermal growth factor | 1.0mg | 160 ± 10 *µ*m |
| | chitosan | 5.0g | |
| | hydroxypropylmethylcellulose 50 cps | 10.0g | |
| | hydroxypropylmethylcellulose 4000cps | 1.0g | |
| | pullulan | 0.05g | |
| | glycerine | 10.0g | |
| | EDTA Disodium | 0.05g | |
| | sodium hydrogen phosphate | 0.142g | |
| | distilled water | proper quantity | |

DWF15 to DWF20 films prepared above were sealed airtight by aluminum packing paper, and then stored at room temperature (25°C) for 3 months, and at cold temperature (2-8°C) for 1 year. The content and activity of epidermal growth factor in each film were measured by time, and shown in the following Table 7 and Fig. 4.

**[Table 7]**

| Classification | 25°C (3 months) | | 2∼8°C (1 year) | |
|---|---|---|---|---|
| | Content (%) | Activity (%) | Content (%) | Activity (%) |
| DWF15 | 77.0 | 68.0 | 87.3 | 87.1 |
| DWF16 | 90.9 | 89.1 | 94.4 | 93.3 |
| DWF17 | 93.7 | 91.2 | 98.1 | 97.2 |
| DWF18 | 92.8 | 91.5 | 96.9 | 97.1 |
| DWF19 | 95.0 | 94.5 | 99.9 | 98.9 |
| DWF20 | 94.5 | 93.9 | 98.2 | 99.1 |

As shown in the above Table 7, it was confirmed that the production rate of impurities of other films than DWF15 which does not comprise stabilizer was very low. Also, the stabilities of DWF16 film comprising amino acid, and DWF17 and DWF18 films comprising antioxidant were a little better. DWF19 film comprising both amino acid and antioxidant, or DWF20 film comprising both antioxidant and buffer showed the most superior stability.

### <Example 5: Animal test>

To confirm the pharmacological effect of epidermal growth factor in the sustained release film formulation of the present invention, the comparison test was conducted by using the epidermal growth factor film prepared with hydroxypropylcellulose as control group, and DWF20 film described in Table 6 of Example 4 prepared by the same method as Example 1 as test group. The conditions of the comparison test were shown in the following Table 8.

**[Table 8]**

| | Control group | Test group |
|---|---|---|
| | General film [25*µ*g/l sheet (20cm²)] | Sustained film formulation of the present invention (DWF20) [50*µ*g/l sheet (20cm²)] |
| Usage and dosage | 1 sheet is attached to the wound site twice a day. | 1 sheet is attached to the wound site once a day. |
| Period of time | 4 days | |
| Area of wound site | about 20cm² | |

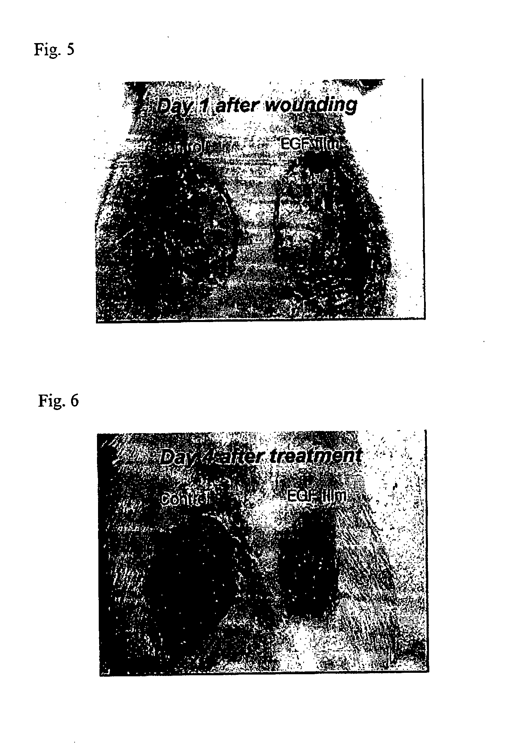

The photograph of wound site after 1 day from the wound was shown in Fig. 5, and the photographs of wound site after 4 days from treating the control and test groups were shown in Fig. 6. As shown in the figures, it was confirmed that the rat's wound treated with the present film comprising epidermal growth factor, chitosan, viscosity modifier, plasticizer, antioxidant and buffer was healed much faster than that treated with the control. The above result was from that EGF was consistently released from the present film, and an effective concentration at wound site was maintained, by treating the present sustained film once, compared with treating the control twice. Also, the treatment effect became better by increasing the antifungal activity through adding chitosan.

### INDUSTRIAL APPLICABILITY

The present invention can maximize wound-healing effect by providing a sustained release film formulation comprising chitosan, viscosity modifier, plasticizer, and stabilizer, in addition to epidermal growth factor as effective ingredient. And, the present sustained film formulation can maintain an effective concentration by once-a-day attachment to wound site unlike ointment/cream formulation or gel formulation which should be applied twice or three times a day. Thus, the present film is not required to be removed from wound site to lower the exposure of wound site, and provide patients and medical workers more convenience.

Also, the present film absorbs exudation, and so is changed to hydrogel to keep wound site humid, and make a favorable condition for wound-healing. And, the present film has superior attachment property to human body, antifungal activity, and stability under cold-storage condition for one or more years. Thus, the present film has more advantages than simple ointment/cream formulation or gel formulation.

## Claims

1. A sustained release film formulation, for healing wound comprising epidermal growth factor; one or more viscosity modifiers selected from the group consisting of hydroxypropylmethylcellulose, gellan gum and pullulan; one or more plasticizers selected from the group consisting of glycerin, propylene glycol, polyethylene glycol, polyvinyl alcohol and polyvinylpyrrolidone; and one or more antioxidants selected from the group consisting of EDTA and vitamin C,
**characterized in that**
the sustained release film formulation further comprises chitosan,
wherein the molecular weight of chitosan is 10 kDa to 150 kDa,
wherein the content of chitosan is 1 to 10 w/w% based on the total weight, and
wherein said hydroxypropylmethylcellulose comprises low-viscosity hydroxypropylmethylcellulose of 10 to 100 cps and high-viscosity hydroxypropylmethylcellulose of 1,000 to 100,000 cps.

2. The film formulation according to claim 1, additionally comprising one or more buffers selected from the group consisting of sodium hydrogen phosphate, sodium hydrogen carbonate and sodium citrate.

3. The film formulation according to claim 1, additionally comprising one or more amino acids selected from the group consisting of L-histidine, L-lysine and L-arginine.

4. The film formulation according to claim 1, wherein the content of epidermal growth factor is 0.1 to 10,000µg/g based on the total weight.

5. The film formulation according to claim 1, wherein the content of viscosity modifier is 0.01 to 20 w/w % based on the total weight.

6. The film formulation according to claim 1, wherein the content of plasticizer is 1 to 40 w/w% based on the total weight.

7. The film formulation according to claim 1, wherein the content of antioxidant is 0.01 to 10 w/w% based on the total weight.

8. The film formulation according to claim 1, wherein the viscosity of film formulation is 1,000 to 150,000 cps.

9. The film formulation according to claim 1, wherein the thickness of film is 0.001 to 1 mm.

10. The film formulation according to any one of claims 1 to 3, additionally comprising foam type of dressing or hydrocolloidal type of dressing.

11. The film formulation according to claim 10, further adding silver or alginate to foam type of dressing or hydrocolloidal type of dressing.

## Patentansprüche

1. Filmformulierung mit anhaltender Freisetzung zur Wundheilung, umfassend einen epidermalen Wachstumsfaktor; einen oder mehrere Viskositätsmodifikator(en), ausgewählt aus der Gruppe bestehend aus Hydroxypropylmethylcellulose, Gellan-Gummi und Pullulan; einen oder mehrere Weichmacher, ausgewählt aus der Gruppe bestehend aus Glycerin, Propylenglykol, Polyethylenglykol, Polyvinylalkohol und Polyvinylpyrrolidon; und ein oder mehrere Antioxidans/Antioxidantien, ausgewählt aus der Gruppe bestehend aus EDTA und Vitamin C,
**dadurch gekennzeichnet, dass**
die Filmformulierung mit anhaltender Freisetzung des Weiteren Chitosan umfasst, wobei das Molekulargewicht von Chitosan 10 kDa bis 150 kDa beträgt,
wobei der Gehalt an Chitosan 1 bis 10 Gew./Gew.-%, basierend auf dem Gesamtgewicht, beträgt, und
wobei diese Hydroxypropylcellulose eine niedrigviskose Hydroxypropylmethylcellulose von 10 bis 100 cPs und eine hochviskose Hydroxypropylmethylcellulose von 1.000 bis 100.000 cPs umfasst.

2. Die Filmformulierung nach Anspruch 1, zusätzlich umfassend einen oder mehrer Puffer, ausgewählt aus der Gruppe bestehend aus Natriumhydrogenphosphat, Natriumhydrogencarbonat und Natriumcitrat.

3. Die Filmformulierung nach Anspruch 1, zusätzlich umfassend eine oder mehrere Aminosäure(n), ausgewählt aus der Gruppe bestehend aus L-Histidin, L-Lysin und L-Arginin.

4. Die Filmformulierung nach Anspruch 1, wobei der Gehalt an epidermalem Wachstumsfaktor 0,1 bis 10.000 µg/g, basierend auf dem Gesamtgewicht, beträgt.

5. Die Filmformulierung nach Anspruch 1, wobei der Gehalt an Viskositätsmodifikator 0,01 bis 20 Gew./Gew.-%, basierend auf dem Gesamtgewicht, beträgt.

6. Die Filmformulierung nach Anspruch 1, wobei der Gehalt an Weichmacher 1 bis 40 Gew./Gew.-%, basierend auf dem Gesamtgewicht, beträgt.

7. Die Filmformulierung nach Anspruch 1, wobei der Gehalt an Antioxidans 0,01 bis 10 Gew./Gew.-%, basierend auf dem Gesamtgewicht, beträgt.

8. Die Filmformulierung nach Anspruch 1, wobei die Viskosität der Filmformulierung 1.000 bis 150.000 cPs beträgt.

9. Die Filmformulierung nach Anspruch 1, wobei die Dicke des Films 0,001 bis 1 mm beträgt.

10. Die Filmformulierung nach einem Beliebigen der Ansprüche 1 bis 3, zusätzlich umfassend einen schaumartigen Verband oder hydrokolloidalartigen Verband.

11. Die Filmformulierung nach Anspruch 10, weiterhin Silber oder Alginat zum schaumartigen Verband oder hydrokolloidalartigen Verband zugefügt.

## Revendications

1. Formulation de film à libération prolongée, destinée à la cicatrisation des plaies comprenant un facteur de croissance épidermique ; un ou plusieurs modificateurs de viscosité choisis dans le groupe constitué par l'hydroxypropylméthylcellulose, la gomme de gellane et le pullulane ; un ou plusieurs plastifiants choisis dans le groupe constitué par la glycérine, le propylène glycol, le polyéthylène glycol, l'alcool polyvinylique et la polyvinylpyrrolidone ; et un ou plusieurs antioxydants choisis dans le groupe constitué par l'EDTA et la vitamine C,
**caractérisée en ce que**
la formulation de film à libération prolongée comprend en outre du chitosane,
dans laquelle le poids moléculaire du chitosane est de 10 kDa à 150 kDa,
dans laquelle la teneur en chitosane est de 1 à 10 % p/p sur la base du poids total, et
dans laquelle ladite hydroxypropylméthylcellulose comprend une hydroxypropylméthylcellulose de viscosité faible de 10 à 100 cps et une hydroxypropylméthylcellulose de viscosité élevée de 1 000 à 100 000 cps.

2. Formulation de film selon la revendication 1,
comprenant en outre un ou plusieurs tampons choisis dans le groupe constitué par l'hydrogénophosphate de sodium, l'hydrogénocarbonate de sodium et le citrate de sodium.

3. Formulation de film selon la revendication 1,
comprenant en outre un ou plusieurs acides aminés choisis dans le groupe constitué par la L-histidine, la L-lysine et la L-arginine.

4. Formulation de film selon la revendication 1,
dans laquelle la teneur en facteur de croissance épidermique est de 0,1 à 10 000 µg/g sur la base du poids total.

5. Formulation de film selon la revendication 1,
dans laquelle la teneur en modificateur de viscosité est de 0,01 à 20 % p/p sur la base du poids total.

6. Formulation de film selon la revendication 1,
dans laquelle la teneur en plastifiant est de 1 à 40 % p/p sur la base du poids total.

7. Formulation de film selon la revendication 1,
dans laquelle la teneur en antioxydant est de 0,01 à 10 % p/p sur la base du poids total.

8. Formulation de film selon la revendication 1,
dans laquelle la viscosité de la formulation de film est de 1 000 à 150 000 cps.

9. Formulation de film selon la revendication 1,
dans laquelle l'épaisseur du film est de 0,001 à 1 mm.

10. Formulation de film selon l'une quelconque des revendications 1 à 3, comprenant en outre un type de pansement mousse ou un type de pansement hydrocolloïdal.

11. Formulation de film selon la revendication 10,
en ajoutant en outre de l'argent ou de l'alginate au type de pansement mousse ou au type de pansement hydrocolloïdal.
